# EUROPÄISCHE PATENTSCHRIFT

(11) **EP 3 355 965 B1**
(45) Veröffentlichungstag und Bekanntmachung des Hinweises auf die Patenterteilung: **23.06.2021**
(21) Anmeldenummer: 16774452.3
(22) Anmeldetag: 26.09.2016
(51) Int. Cl.: A61M 5/20, A61M 5/315, A61M 5/24

(54) **AUTOINJEKTOR MIT DOSIERVORRICHTUNG**
AUTOINJECTOR WITH METERING DEVICE
AUTO-INJECTEUR COMPRENANT UN DOSEUR

(30) Priorität: 30.09.2015 DE 202015006845 U
(43) Veröffentlichungstag der Anmeldung: 08.08.2018
(73) Patentinhaber: Haselmeier AG, 9001 St. Gallen (CH)
(72) Erfinder: KEITEL, Joachim, 73728 Esslingen (DE)
(74) Vertreter: Manitz Finsterwald Patent- und Rechtsanwaltspartnerschaft mbB
(86) Internationale Anmeldenummer: PCT/EP2016/001597
(87) Internationale Veröffentlichungsnummer: WO 2017/054914

(56) Entgegenhaltungen:
- EP-A1- 0 525 525
- EP-A2- 0 268 191
- WO-A1-2004/078241
- WO-A1-2007/071080
- WO-A1-2013/117332
- WO-A1-2015/007816
- WO-A1-2015/117747

## Beschreibung

Die Erfindung betrifft ein Injektionsgerät der im Oberbegriff des Anspruchs 1 angegebenen Gattung.

Aus der WO 2013/117332 A1 ist ein Injektionsgerät der gattungsgemäßen Art bekannt. Das Injektionsgerät besitzt ein Dosierorgan, das beim Einstellen einer auszupressenden Dosis an Injektionsflüssigkeit gegenüber dem Gehäuse gedreht wird. Über eine erste Gewindeverbindung bewegt sich eine Injektionshülse in distale Richtung. Dabei bewegt sich die Injektionshülse in distaler Richtung aus dem Gehäuse. Der an der Injektionshülse gelagerte Bedienknopf bewegt sich mit der Injektionshülse in distale Richtung. Beim Auspressen einer eingestellten Dosis an Injektionsflüssigkeit drückt der Bediener das Bedienelement in proximale Richtung und verschiebt dadurch die Injektionshülse in proximaler Richtung. Dadurch dreht sich das Dosierorgan aufgrund der ersten Gewindeverbindung in Gegenrichtung gegenüber dem Gehäuse und bewirkt das Auspressen von Injektionsflüssigkeit aus einem Behälter.

Die WO 2007/071080 A1 zeigt ein Injektionsgerät mit einem drehbaren und axial beweglichen Dosier- und Anzeigeglied, das über eine Gewindeverbindung mit dem Gehäuse verbunden ist. Das Dosier- und Anzeigeglied wird beim Auspressen von Injektionsflüssigkeit von einer Feder in Richtung zur Injektionsnadel bewegt und presst dabei Injektionsflüssigkeit aus.

Die WO 2015/117747 A1 zeigt ein Injektionsgerät mit einem Einstellteil, das über eine Gewindeverbindung mit dem Gehäuse verbunden ist und sich beim Einstellen von Injektionsflüssigkeit dreht und in distaler Richtung bewegt und beim Auspressen von Injektionsflüssigkeit in einer zweiten Drehrichtung dreht und in proximale Richtung bewegt.

Die EP 0 268 191 A2 zeigt ein Injektionsgerät mit einer vom Bediener spannbaren Federanordnung, die vom Bediener auslösbar ist und eine Bewegung eines Stößels in proximaler Richtung und dadurch eine Bewegung der Injektionsnadel sowie ein Auspressen von zu injizierender Flüssigkeit aus dem Behälter bewirkt.

Die EP 0 525 525 A1 zeigt ein Injektionsgerät mit einem Verstellglied, das über eine Gewindeverbindung mit einer Gewindestange verbunden ist. Beim Auspressen von Injektionsflüssigkeit dreht sich das Verstellglied und bewegt dadurch die Gewindestange in axialer Richtung.

Aus der WO 2015/007816 A1 geht eine Anordnung zur Verabreichung eines Medikaments hervor, bei der beim Einstellen einer Dosis ein Antriebsglied über eine Gewindeverbindung zu einem Dosierglied axial bewegt wird. Beim Einstellen der Dosis ist das Antriebsglied mit der Kolbenstange nicht verbunden. Beim Einstellen der Dosis wird eine Torsionsfeder gespannt. Zum Auspressen von Injektionsflüssigkeit wird das Antriebsglied in radialer Richtung zum Dosierkolben bewegt und mit diesem in Eingriff gebracht. Aufgrund der in der Torsionsfeder gespeicherten Kraft wird das Dosierglied zurückgedreht und dabei das Antriebsglied axial bewegt. Aufgrund des Eingriffs mit dem Dosierkolben wird auch der Dosierkolben axial bewegt und presst dabei Injektionsflüssigkeit aus.

Der Erfindung liegt die Aufgabe zugrunde, ein Injektionsgerät der gattungsgemäßen Art zu schaffen, bei dem die Injektion automatisch erfolgt.

Diese Aufgabe wird durch ein Inj ektionsgerät mit den Merkmalen des Anspruchs 1 gelöst.

Es ist vorgesehen, dass das Injektionsgerät eine Feder besitzt, die sich mit einem ersten Ende gegenüber der Injektionshülse und mit einem zweiten Ende gegenüber dem Gehäuse abstützt. Die Feder wirkt demnach zwischen zwei beim Einstellen einer auszupressenden Dosis an Injektionsflüssigkeit in Richtung der Längsmittelachse zu einander bewegten Bauteilen. Beim Auspressen von Injektionsflüssigkeit ist vorgesehen, dass die Feder die Injektionshülse in proximale Richtung bewegt und dadurch das Auspressen der eingestellten Dosis von Injektionsflüssigkeit aus einem Behälter bewirkt. Die Gewindeverbindung der Injektionshülse kann so ausgelegt werden, dass die Injektionshülse einen vergleichsweise großen axialen Weg zurücklegt und ein ausreichend großer Spannweg zum Spannen der Feder gewährleistet werden kann. Der Weg, um den sich die Injektionshülse in axialer Richtung bewegt, ist über die Auslegung der ersten Gewindeverbindung einstellbar, so dass auf einfache Weise eine Anpassung an den gewünschten Spannweg ermöglicht ist.

Vorteilhaft ist die Feder als Druckfeder ausgebildet. Die Feder ist dabei insbesondere eine Schraubendruckfeder. Auch eine Ausbildung der Feder als Zugfeder, insbesondere als Schraubenzugfeder kann jedoch vorteilhaft sein. Ein einfacher Aufbau ergibt sich, wenn die Feder sich mit ihrem ersten Ende unmittelbar an der Injektionshülse und mit ihrem zweiten Ende unmittelbar am Gehäuse abstützt. Die Feder wirkt demnach unmittelbar zwischen Injektionshülse und Gehäuse. Es kann jedoch auch vorteilhaft sein, dass die Feder sich an einem mit der Injektionshülse und/oder mit dem Gehäuse verbundenen Bauteil abstützt.

Ein kompakter Aufbau wird erreicht, wenn das Dosierorgan radial innerhalb der Injektionshülse angeordnet ist. Vorteilhaft ist die Feder am Außenumfang des Dosierorgans und mindestens teilweise in einem zwischen Dosierorgan und Injektionshülse gebildeten Ringraum angeordnet.

Um auf einfache Weise ein Ablesen einer eingestellten Dosis zu erlauben, ist vorgesehen, dass das Gehäuse ein Sichtfenster besitzt und dass die Injektionshülse eine Öffnung aufweist, die in Überdeckung mit dem Sichtfenster liegt und durch die eine am Außenumfang des Dosierorgans angeordnete Skala sichtbar ist. Um auch bei großem axialem Weg des Injektionsgeräts und mehreren Umdrehungen des Dosierorgans bis zur maximal einstellbaren Dosis eine eindeutige Anzeige der eingestellten Dosis zu erreichen, ist vorgesehen, dass die Injektionshülse einen Abschnitt besitzt, der bei maximaler eingestellter Dosis den proximalen Bereich des Sichtfensters abdeckt. Um eine geringe Baulänge des Injektionsgeräts zu erreichen, ist vorteilhaft vorgesehen, dass der Abschnitt, der den proximalen Bereich des Sichtfensters abdeckt, an einem in proximale Richtung ragenden Steg der Injektionshülse ausgebildet ist. Der Steg erstreckt sich dabei vorteilhaft nur im Bereich des Sichtfensters, also nicht über den gesamten Umfang des Injektionsgeräts. Es ist vorgesehen, dass das Injektionsgerät einen Behälter mit Injektionsflüssigkeit umfasst. In Nullstellung des Injektionsgeräts, also wenn keine auszupressende Menge an Injektionsflüssigkeit eingestellt ist, ragt der Steg vorteilhaft in den Bereich des Behälters. Vorteilhaft ist in radialer Richtung zwischen dem Behälter und einem oberen Gehäuseteil des Injektionsgeräts eine Tasche gebildet, in die der Steg in Nullstellung ragt. Dadurch, dass sich der Steg und der Behälter in axialer Richtung überlappen, kann eine vergleichsweise geringe Baulänge des Injektionsgeräts erreicht werden.

Das Gehäuse besitzt vorteilhaft eine Gehäusewand, an der ein Drehlager für das Dosierorgan ausgebildet ist. Die Gehäusewand verläuft vorteilhaft etwa senkrecht zur Längsmittelachse. Der Steg ragt in Nullstellung vorteilhaft durch eine Durchtrittsöffnung in der Gehäusewand auf die proximale Seite der Gehäusewand. Vorteilhaft ist auf der proximalen Seite der Gehäusewand der Behälter angeordnet und die Tasche für den Steg ausgebildet.

Es ist vorgesehen, dass die Injektionshülse in jeder Stellung des Injektionsgeräts vollständig im Gehäuse des Injektionsgeräts angeordnet ist. Eine Bewegung der Injektionshülse in distaler Richtung aus dem Gehäuse 2 hinaus ist vorteilhaft nicht vorgesehen.

Das Injektionsgerät besitzt vorteilhaft ein Bedienelement, das beim Einstellen der auszupressenden Dosis an Injektionsflüssigkeit über eine erste Kupplung drehfest mit dem Dosierorgan verbunden ist und dass beim Auspressen einer eingestellten Dosis an Injektionsflüssigkeit über eine zweite Kupplung drehfest mit dem Gehäuse verbunden und gegenüber dem Dosierorgan drehbar ist. Das Bedienelement befindet sich beim Einstellen der Dosis vorteilhaft in einer distalen Endlage. Das Bedienelement wird beim Einstellen der Dosis demnach nicht mit der Injektionshülse in distaler Richtung bewegt. Zum Lösen der ersten Kupplung ist das Bedienelement vorteilhaft in proximaler Richtung zu bewegen. Das Lösen der ersten Kupplung entspricht dabei dem Auslösen der Injektion, da bei gelöster erster Kupplung das Dosierorgan gegenüber dem Bedienelement und damit gegenüber dem Gehäuse drehbar ist und die Feder die Injektionshülse in proximale Richtung bewegen und dabei das Dosierorgan drehen kann.

Zum Auspressen von Injektionsflüssigkeit aus dem Behälter ist ein Dosierkolben vorgesehen, der über eine zweite Gewindeverbindung mit dem Dosierorgan verbunden ist. Beim Einstellen der auszupressenden Menge an Injektionsflüssigkeit ist der Dosierkolben drehfest mit dem Dosierorgan verbunden und dreht sich mit dem Dosierorgan. Beim Auspressen einer auszupressenden Menge an Injektionsflüssigkeit ist der Dosierkolben drehfest mit dem Gehäuse verbunden und bewegt sich aufgrund der zweiten Gewindeverbindung in proximale Richtung. Dadurch wird ein einfacher Aufbau des Injektionsgeräts erreicht.

Ausführungsbeispiele der Erfindung werden im Folgenden anhand der Zeichnung erläutert. Es zeigen:
- Fig. 1: eine Seitenansicht eines Injektionsgeräts in Nullstellung,
- Fig. 2: einen Schnitt entlang der Linie II-II in Fig. 1,
- Fig. 3: eine Seitenansicht des Injektionsgeräts aus Fig. 1 in Maximalstellung,
- Fig. 4: einen Schnitt entlang der Linie IV-IV in Fig. 1,
- Fig. 5: eine perspektivische Darstellung des Bedienelements des Injektionsgeräts aus den Fig. 1 bis 4,
- Fig. 6: eine Seitenansicht des Bedienelements aus Fig. 5,
- Fig. 7: einen Schnitt entlang der Linie VII-VII in Fig. 6,
- Fig. 8: eine Ansicht des Bedienelements in Richtung des Pfeils VIII in Fig. 6,
- Fig. 9: eine schematische Darstellung eines Raststegs des Bedienelements,
- Fig. 10: eine schematische perspektivische Darstellung des Bedienelements in zwei Einzelteilen,
- Fig. 11: eine Seitenansicht der beiden Einzelteile des Bedienelements,
- Fig. 12: eine Seitenansicht des Mitnehmers des Injektionsgeräts,
- Fig. 13: einen Schnitt entlang der Linie XIII-XIII in Fig. 12,
- Fig. 14: einen Schnitt entlang der Linie XIV-XIV in Fig. 12,
- Fig. 15: einen Schnitt entlang der Linie XV-XV in Fig. 12,
- Fig. 16: einen Schnitt entlang der Linie XVI-XVI in Fig. 12,
- Fig. 17: eine Seitenansicht des Verbindungselements des Injektionsgeräts,
- Fig. 18: einen Schnitt entlang der Linie XVIII-XVIII in Fig. 17,
- Fig. 19: eine Seitenansicht einer Kolbenstange des Injektionsgeräts,
- Fig. 20: einen Schnitt entlang der Linie XX-XX in Fig. 19,
- Fig. 21: einen Schnitt entlang der Linie XXI-XXI in Fig. 19,
- Fig. 22: eine Seitenansicht von Bedienelement und Mitnehmer in einer Kupplungsposition,
- Fig. 23: einen Schnitt entlang der Linie XXIII-XXIII in Fig. 22,
- Fig. 24: eine Seitenansicht von Bedienelement und Mitnehmer in einer ersten Relativposition,
- Fig. 25: einen Schnitt entlang der Linie XXV-XXV in Fig. 24,
- Fig. 26: eine Seitenansicht von Bedienelement und Mitnehmer in einer zweiten Relativposition,
- Fig. 27: einen Schnitt entlang der Linie XXVII-XXVII in Fig. 26,
- Fig. 28: eine Seitenansicht von Bedienelement und Mitnehmer in einer dritten Relativposition,
- Fig. 29: einen Schnitt entlang der Linie XIX-XIX in Fig. 28,
- Fig. 30: eine Seitenansicht eines Dosierorgans des Injektionsgeräts,
- Fig. 31: einen Schnitt entlang der Linie XXXI-XXXI in Fig. 30,
- Fig. 32: eine Seitenansicht einer Injektionshülse des Injektionsgeräts,
- Fig. 33: einen Schnitt entlang der Linie XXXIII-XXXIII in Fig. 32,
- Fig. 34: einen Schnitt entlang der Linie XXXIV-XXXIV in Fig. 32,
- Fig. 35: eine Seitenansicht eines oberen Gehäuseteils des Injektionsgeräts,
- Fig. 36: einen Schnitt entlang der Linie XXXVI-XXXVI in Fig. 35,
- Fig. 37: einen Schnitt entlang der Linie XXXVII-XXXVII in Fig. 35,
- Fig. 38: eine Seitenansicht in Richtung des Pfeils XXXVIII in Fig. 36,
- Fig. 39: eine Seitenansicht eines weiteren Injektionsgeräts in Nullstellung,
- Fig. 40: einen Schnitt entlang der Linie XL-XL in Fig. 39,
- Fig. 41: den Ausschnitt XLI aus Fig. 40 in vergrößerter Darstellung,
- Fig. 42: einen Schnitt entlang der Linie XLII-XLII in Fig. 41,
- Fig. 43: einen Schnitt entlang der Linie XLIII-XLIII in Fig. 41,
- Fig. 44: eine Seitenansicht des Injektionsgeräts in einer Endstellung nach dem Auspressen einer eingestellten Dosis an Injektionsflüssigkeit,
- Fig. 45: einen Schnitt entlang der Linie XLV-XLV in Fig. 44,
- Fig. 46: den Ausschnitt XLVI aus Fig. 45 in vergrößerter Darstellung,
- Fig. 47: einen Schnitt entlang der Linie XLVII-XLVII in Fig. 46,
- Fig. 48: einen Schnitt entlang der Linie XLVIII-XLVIII in Fig. 46,
- Fig. 49 und Fig. 50: perspektivische Darstellungen des Mitnehmers des Injektionsgeräts,
- Fig. 51: eine Seitenansicht des Mitnehmers,
- Fig. 52: einen Schnitt entlang der Linie LII-LII in Fig. 51,
- Fig. 53: einen Schnitt entlang der Linie LIII-LIII in Fig. 51,
- Fig. 54: einen Schnitt entlang der Linie LIV-LIV in Fig. 51.

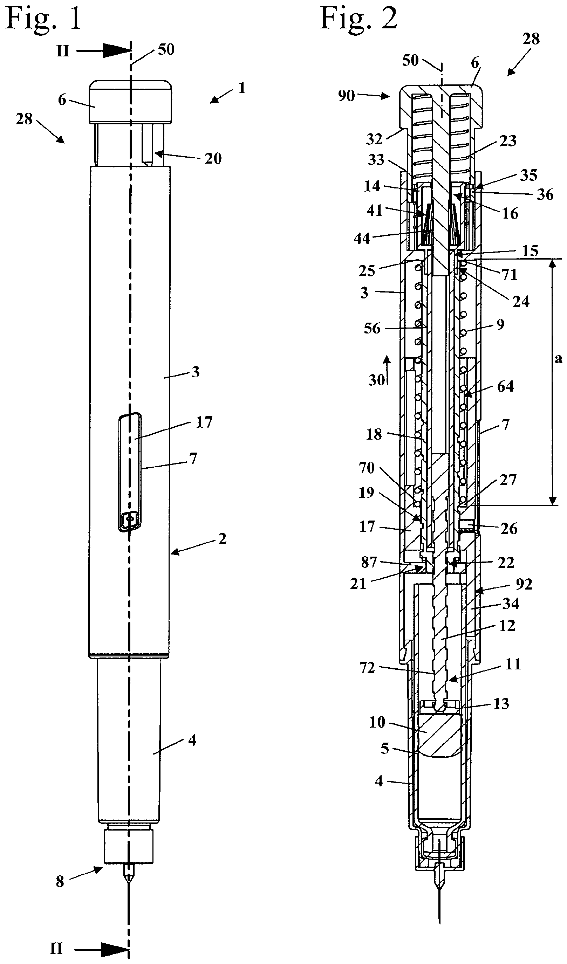

Fig. 1 zeigt als Ausführungsbeispiel für ein mechanisches Injektionsgerät, bei dem das Auspressen einer Dosis von Injektionsflüssigkeit automatisch erfolgt, ein Injektionsgerät 1. Das Injektionsgerät 1 besitzt ein Gehäuse 2, das ein oberes Gehäuseteil 3 und einen am oberen Gehäuseteil 3 festgelegten Halter 4 umfasst. Der Halter 4 ist an der proximalen Seite des oberen Gehäuseteils 3 angeordnet. An der proximalen Seite des Halters 4 ist eine Injektionsnadel 8 festgelegt. An der distalen Seite des Injektionsgeräts 1 ist ein Bedienelement 6 angeordnet. Das Bedienelement 6 ist über eine Kupplung 20 mit dem oberen Gehäuseteil 3 drehfest verbindbar. Das obere Gehäuseteil 3 besitzt ein Sichtfenster 7, das vorteilhaft aus durchsichtigem Material besteht, so dass durch das Sichtfenster 7 eine im oberen Gehäuseteil 3 angeordnete Injektionshülse 17 sichtbar ist. Das Injektionsgerät 1 besitzt eine Längsmittelachse 50, die in Längsrichtung des Gehäuses 2 des Injektionsgeräts 1 verläuft.

Das distale Ende des Injektionsgeräts 1 ist das einer am Injektionsgerät 1 gehaltenen Injektionsnadel 8 abgewandt liegende Ende. Mit "proximal" wird die Seite des Injektionsgeräts 1 bezeichnet, die bei einer Injektion der Einstichstelle zugewandt liegt, und mit "distal" die Seite, die der Einstichstelle abgewandt liegt. Die proximale Richtung bezeichnet die Injektionsrichtung, also die Richtung zur Injektionsnadel 8 hin bzw. die Richtung, in der die Injektionsflüssigkeit aus einem Behälter ausgepresst wird. Die distale Richtung bezeichnet die entgegengesetzte Richtung, also von der Injektionsnadel 8 weg.

Die Fig. 1 und 2 zeigen das Injektionsgerät 1 in einer Nullstellung 28, in der keine Dosis von Injektionsflüssigkeit eingestellt ist. Das Bedienelement 6 befindet sich in seiner distalen Endlage 90. Wie Fig. 2 zeigt, ist im Halter 4 ein Behälter 5 mit Injektionsflüssigkeit angeordnet. Im Behälter 5 ist ein Stopfen 10 angeordnet, an dem eine Kolbenscheibe 13 eines Dosierkolbens 11 anliegt. Der Dosierkolben 11 umfasst außerdem eine Kolbenstange 12, die ein Außengewinde 72 trägt.

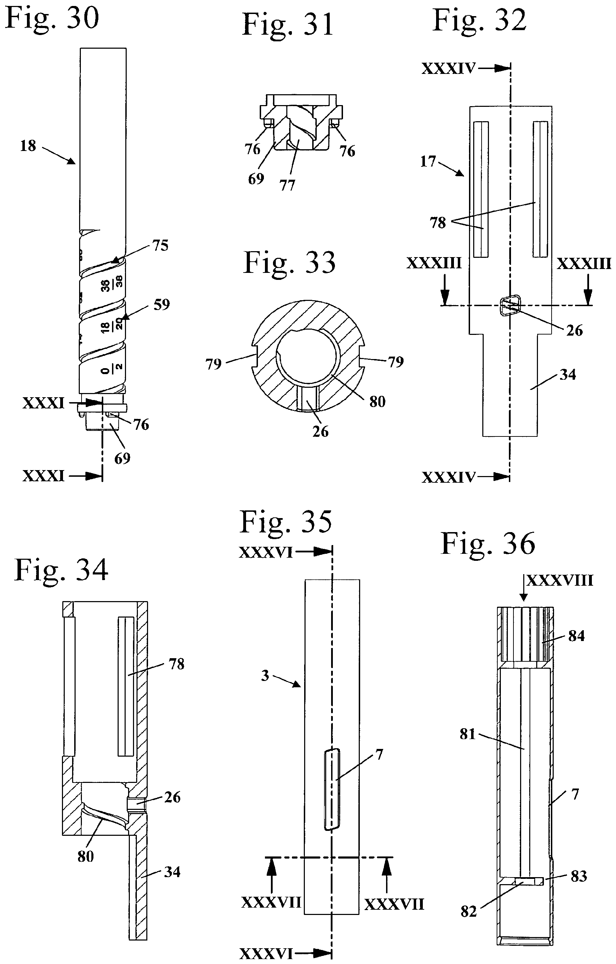

Die Außenseite der Injektionshülse 17 ist durch das Sichtfenster 7 des oberen Gehäuseteils 3 sichtbar. Die Injektionshülse 17 besitzt eine Öffnung 26, durch die der Außenumfang eines radial innerhalb der Injektionshülse 17 angeordneten Dosierorgans 18 sichtbar ist. Das Dosierorganl8, das auch als Skalenrohr bezeichnet werden kann, trägt an seinem Außenumfang eine in Fig. 30 gezeigte Skala 59, die durch das Sichtfenster 7 und die Öffnung 26 für den Bediener sichtbar ist und die eingestellte Dosis von auszupressender Injektionsflüssigkeit anzeigt.

Die Injektionshülse 17 ist im oberen Gehäuseteil 3 in Richtung der Längsmittelsachse 50 verschiebbar und gegenüber dem oberen Gehäuseteil 3 drehfest gehalten. Die Injektionshülse 17 ist dabei in jeder Stellung des Injektionsgeräts 1 vollständig innerhalb des Gehäuses 2, nämlich innerhalb des oberen Gehäuseteils 3, angeordnet. Das Dosierorgan 18 und die Injektionshülse 17 sind über eine erste Gewindeverbindung 19 miteinander verbunden. Das Dosierorgan 18 ist an einem Drehlager 21 drehbar im oberen Gehäuseteil 3 gelagert und axial unverschiebbar im oberen Gehäuseteil 3 gehalten. Das Dosierorgan 18 ist über eine zweite Gewindeverbindung 22 mit dem Außengewinde 72 der Kolbenstange 12 verbunden.

Im oberen Gehäuseteil 3 ist ein Mitnehmer 14 gelagert. Der Mitnehmer 14 ist über eine drehfeste Verbindung 24 drehfest mit dem Dosierorgan 18 verbunden. Die drehfeste Verbindung 24 kann eine Pressverbindung sein. Es kann jedoch auch vorgesehen sein, dass die drehfeste Verbindung 24 eine formschlüssige Verbindung ist. Der Mitnehmer 14 ist an einem im oberen Gehäuseteil 3 ausgebildeten Drehlager 15 drehbar gelagert. Das Drehlager 15 wird durch einen Rand des oberen Gehäuseteils 3 gebildet.

Wie Fig. 2 auch zeigt, ist im oberen Gehäuseteil 3 eine Feder 9 angeordnet, die als Druckfeder, nämlich als Schraubendruckfeder ausgebildet ist. Die Feder 9 stützt sich mit einem ersten Ende 70 an einem Anlagerand 27 der Injektionshülse 17 ab und mit einem zweiten Ende 71 an einem Anlagerand 25 des oberen Gehäuseteils 3. Am Anlagerand 25 ist auch das Drehlager 15 für den Mitnehmer 14 ausgebildet. Die Feder 9 ist radial außerhalb des Dosierorgans 18 angeordnet und ragt in der in Fig. 2 gezeigten Nullstellung 28 mit ihrem proximalen Bereich in einen zwischen Injektionshülse 17 und Dosierorgan 18 gebildeten Ringraum 64.

Das Bedienelement 6 ist über ein Verbindungselement 56, das als Hülse ausgebildet ist, drehfest mit der Kolbenstange 12 verbunden. Das Bedienelement 6 stützt sich über eine Feder 23, die als Schraubendruckfeder ausgebildet ist, gegenüber dem oberen Gehäuseteil 3 ab. Die Feder 23, die das Bedienelement 6 in distale Richtung drückt, hat auf die Geschwindigkeit der Injektion keinen Einfluss. Die Feder 23 ist lediglich so ausgelegt, dass der Bediener das Bedienelement 6 mit angenehmer Kraft betätigen kann. Am Bedienelement 6 ist ein Absatz 32 ausgebildet, der bei in proximale Richtung gedrücktem Bedienelement 6 mit einem Rand 33 des oberen Gehäuseteils 3 zusammenwirkt und mit diesem einen Anschlag bildet, der die proximale Position des Bedienelements 6 begrenzt. Zur Festlegung der distalen Endlage 90 des Bedienelements 6 ist vorteilhaft ein weiterer Anschlag vorgesehen, der beispielsweise am Mitnehmer 14 ausgebildet sein kann. Zwischen dem Bedienelement 6 und dem oberen Gehäuseteil 3 wirkt eine Rasteinrichtung 35, die mehrere Rastarme 36 umfasst, von denen in Fig. 2 einer sichtbar ist. Das Bedienelement 6 ist in der in den Fig. 1 und 2 gezeigten Nullstellung 28 über eine Kupplung 16 drehfest mit dem Mitnehmer 14 gekoppelt. Zwischen dem Bedienelement 6 und dem Mitnehmer 14 ist außerdem eine Einstellvorrichtung 41 gebildet, die eine Vielzahl von Rastvertiefungen 44 im Mitnehmer 14 umfasst.

Bei unbetätigtem Bedienelement 6 drückt die Feder 23 das Bedienelement 6 in seine distale Endlage 90, in der die Kupplung 20 geöffnet und das Bedienelement 6 gegenüber dem Gehäuse 2 drehbar ist. Zum Einstellen einer auszupressenden Menge an Injektionsflüssigkeit dreht der Bediener das Bedienelement 6 um die Längsmittelachse 50. Dabei dreht sich der über die Kupplung 16 drehfest mit dem Bedienelement 6 verbundene Mitnehmer 14 mit. Der Mitnehmer 14 ist über eine drehfeste Verbindung 24 mit dem Dosierorgan 18 verbunden, das sich ebenfalls mitdreht. Die Kolbenstange 12 ist über das Verbindungselement 56 drehfest mit dem Bedienelement 6 verbunden und dreht sich ebenfalls mit. Aufgrund der ersten Gewindeverbindung 19 und der drehfesten Fixierung der Injektionshülse 17 im oberen Gehäuseteil 3 wird die Injektionshülse 17 bei der Drehbewegung des Dosierorgans 18 in distale Richtung 30 bewegt. Dabei bewegt sich die Injektionshülse 17 mit ihrem Anlagerand 27 auf den Anlagerand 25 der Feder 9 zu, wodurch die Feder 9 gespannt wird. Der Anlagerand 25 des Gehäuses 2 kann dabei einen Anschlag für die distale Position der Injektionshülse 17, also auch für die maximal einstellbare Dosis, bilden. Die axiale Position des Bedienelements 6 verändert sich beim Einstellen der auszupressenden Dosis von Injektionsflüssigkeit nicht. Aufgrund der Bewegung der Injektionshülse 17 in distale Richtung hat sich die Länge des Ringraums 64 verkürzt.

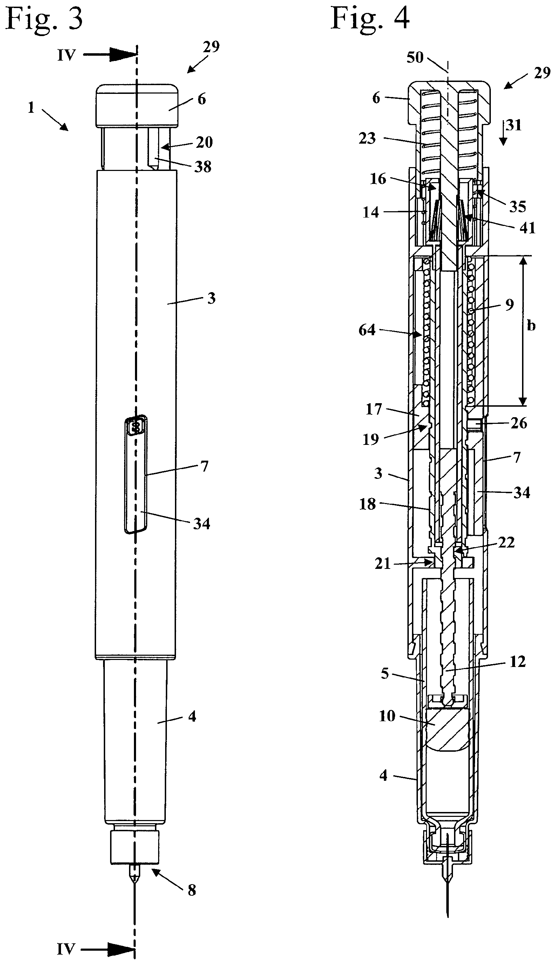

Die Fig. 3 und 4 zeigen das Injektionsgerät 1 in einer Maximalstellung 29, in der die maximale Dosis eingestellt ist. Die Länge der Feder 9 hat sich von der in Fig. 2 gezeigten ungespannten Länge a auf die in Fig. 4 gezeigte gespannte Länge b verkürzt. Es kann auch vorgesehen sein, dass die Feder 9 auch in Nullstellung 28 vorgespannt ist. Die Injektionshülse 17 kann am Anlagerand 25 anliegen. Im Ausführungsbeispiel ist die Feder 9 in Maximalstellung 29 vollständig im Ringraum 64 angeordnet. Wie Fig. 3 zeigt, ist durch das Sichtfenster 7 in Maximalstellung 29 die maximale Dosis sichtbar. Die Injektionshülse 17 besitzt einen Steg 34, der in proximale Richtung ragt und den durch das Sichtfenster 7 sichtbaren Bereich des Dosierorgans 18, der nicht die eingestellte Dosis anzeigt, abdeckt. In radialer Richtung zwischen dem Behälter 5 und dem oberen Gehäuseteil 3 ist eine Tasche 92 gebildet, in die der Steg 34 in Nullstellung 28 ragt. Der Steg 34 ragt dabei durch eine Gehäusewand 87 des oberen Gehäuseteils 3 auf die proximale Seite der Gehäusewand 87. An der Gehäusewand 87 ist das Drehlager 21 für das Dosierorgan 18 ausgebildet. Im Bereich der Gehäusewand 87 ist auch die Gewindeverbindung 22 zwischen Kolbenstange 12 und Dosierorgan 18 angeordnet.

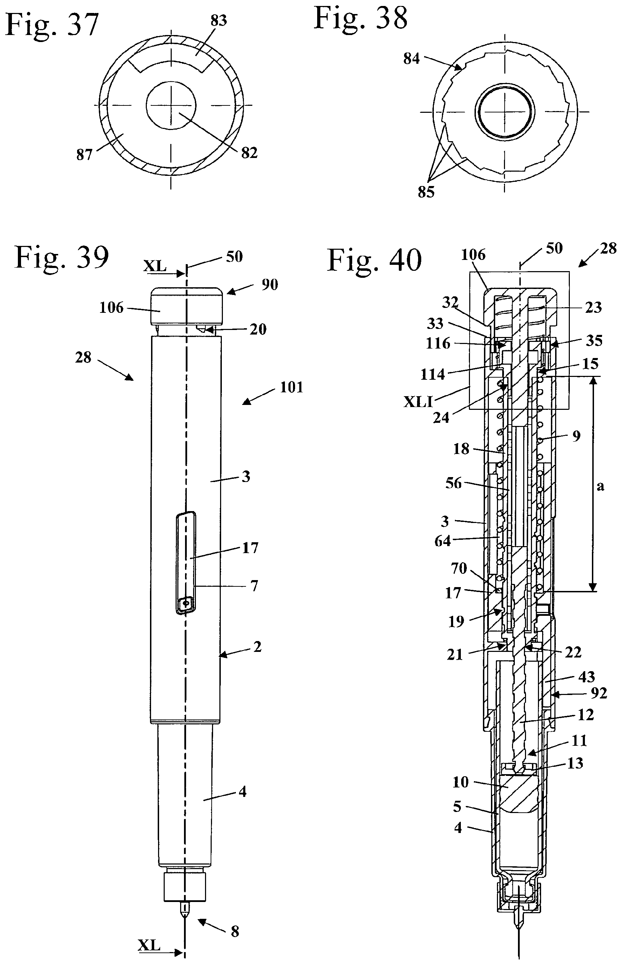

Zum Auspressen einer eingestellten Menge an Injektionsflüssigkeit drückt der Bediener das Bedienelement 6 in proximale Richtung 31. Dadurch gelangen Stege 38 der Kupplung 20 (Fig. 1) in Eingriff mit Rastelementen 85 der Rasteinrichtung 35 (Fig. 38). Dadurch wird das Bedienelement 6 gegenüber dem oberen Gehäuseteil 3 drehfest fixiert. Gleichzeitig wird die Kupplung 16 aufgrund der axialen Relativbewegung des Bedienelements 6 gegenüber dem Mitnehmer 14 mindestens teilweise gelöst, so dass sich der Mitnehmer 14 gemeinsam mit dem Dosierorgan 18 um die Längsmittelachse 50 drehen kann. Die Drehbewegung erfolgt aufgrund der von der gespannten Feder 9 auf die Injektionshülse 17 ausgeübten axialen Kraft, die eine Drehung des Dosierorgans 18 bewirkt. Die Drehung erfolgt aufgrund der Gewindeverbindung 19 und der drehfesten Führung der Injektionshülse 17 im Gehäuseteil 3. Die Kolbenstange 12 ist über das Verbindungselement 56 und das Bedienelement 6 drehfest mit dem oberen Gehäuseteil 3 verbunden. Die zweite Gewindeverbindung 22 bewirkt bei der Drehung des Dosierorgans 18 deshalb eine Bewegung der Kolbenstange 12 in proximaler Richtung 31. Dadurch wird die eingestellte Menge an Injektionsflüssigkeit aus dem Behälter 5 ausgepresst.

Die Injektion erfolgt nach dem Lösen der Kupplung 16 aufgrund der in der Feder 9 gespeicherten Kraft automatisch. Die Feder 9 ist so ausgelegt, dass die in der Feder 9 gespeicherte Kraft ausreicht, um den Widerstand des Stopfens 10 zu überwinden und Injektionsflüssigkeit aus dem Behälter 5 auszupressen. Zur Einstellung der Injektionsgeschwindigkeit besitzt das Injektionsgerät 1 die Einstellvorrichtung 41. Die Einstellvorrichtung 41 beeinflusst das Drehmoment, das zur Drehung des Mitnehmers 14 gegenüber dem Bedienelement 6 benötigt wird. Das benötigte Drehmoment ist dabei abhängig von der axialen Position des Bedienelements 6 gegenüber dem oberen Gehäuseteil 3 und dem Mitnehmer 14. Dies wird im Folgenden noch näher erläutert.

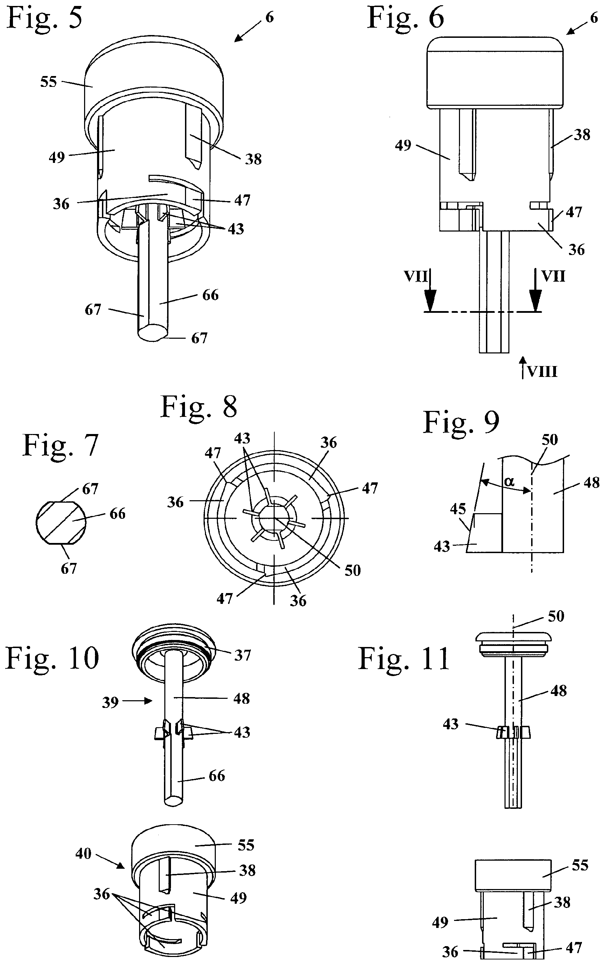

Die Fig. 5 bis 11 zeigen den Aufbau des Bedienelements 6 im Einzelnen. Das Bedienelement 6 besitzt einen Bedienabschnitt 55, der aus dem oberen Gehäuseteil 3 ragt und an dem der Bediener das Bedienelement 6 drehen oder in proximale Richtung 31 verschieben kann. Das Bedienelement 6 besitzt einen in den Fig. 5 und 6 gezeigten Hülsenabschnitt 49, der in Nullstellung 28 und Maximalstellung 29 teilweise aus dem oberen Gehäuseteil 32 ragt und an dem die Stege 38 der Kupplung 20 fixiert sind. An seinem proximalen Ende trägt der Hülsenabschnitt 49 im Ausführungsbeispiel insgesamt drei Rastarme 36, die an ihrem freien Ende jeweils ein Rastelement 47 tragen. Dies ist auch in Fig. 8 gezeigt. Die nach außen ragenden Rastelemente 47 sind mit den Rastarmen 36 radial nach innen beweglich gelagert.

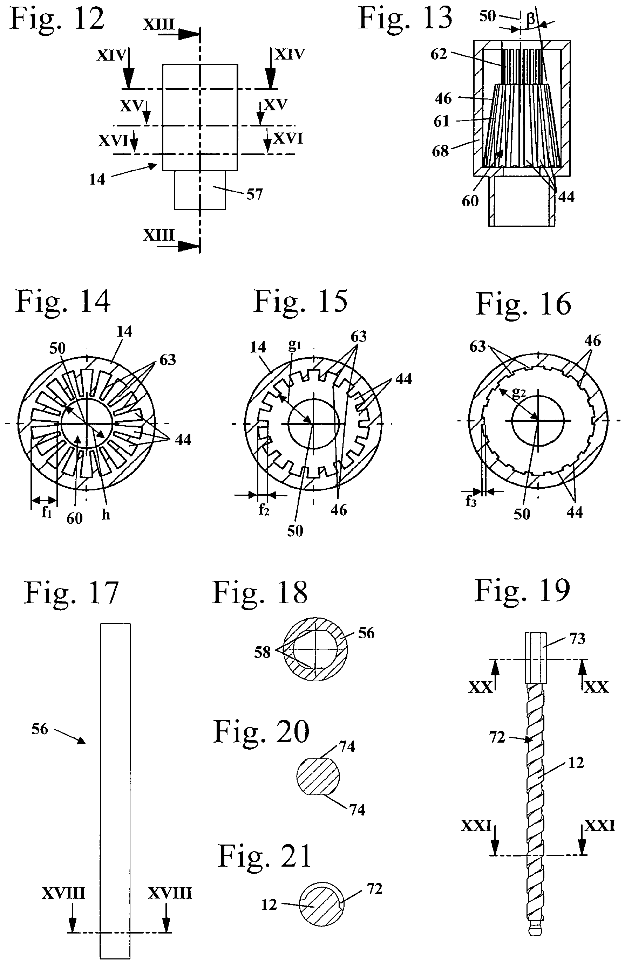

Wie Fig. 5 auch zeigt, besitzt das Bedienelement 6 einen Verbindungsstutzen 66, der an seinem Außenumfang zwei Abflachungen 67 zur drehfesten Verbindung mit dem Verbindungselement 56 trägt. Wie die Fig. 17 und 18 zeigen, besitzt das Verbindungselement 56, das hülsenförmig ausgebildet ist, an seiner Innenseite entsprechende Abflachungen 58, die mit den Abflachungen 67 des Verbindungsstutzens 66 zusammenwirken und das Bedienelement 6 und das Verbindungselement 56 dadurch drehfest miteinander verbinden. Wie die Fig. 19 und 20 zeigen, trägt die Kolbenstange 12 an einem distalen Endabschnitt 73 entsprechende Abflachungen 74 zur drehfesten Verbindung von Kolbenstange 12 und Verbindungselement 56. In den Fig. 19 und 21 ist auch das Außengewinde 72 der Kolbenstange 12 gezeigt.

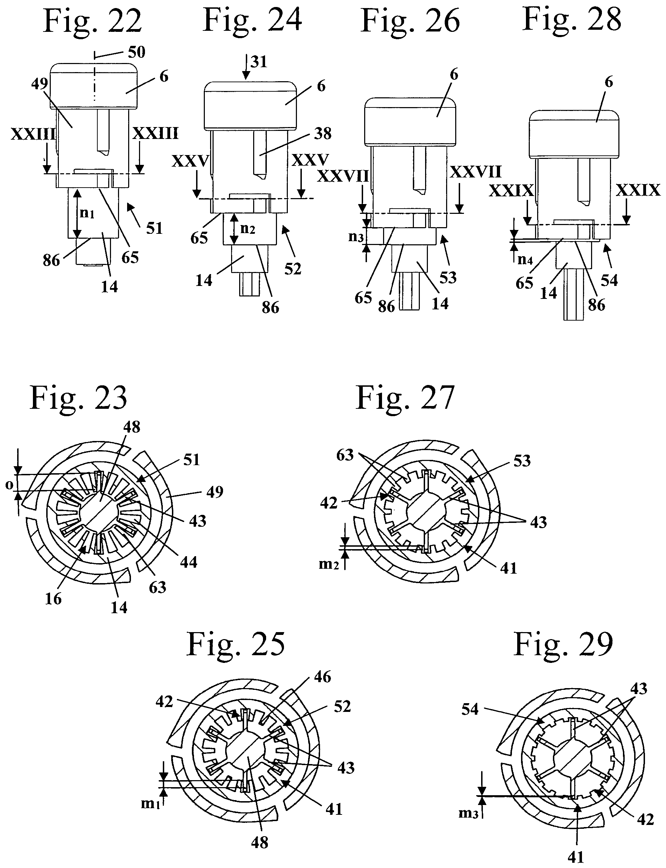

Wie die Fig. 5 und 8 zeigen, besitzt das Bedienelement 6 im Ausführungsbeispiel sechs bezogen auf die Längsmittelachse 50 radial nach außen ragende Raststege 43, die mit den Rastvertiefungen 44 des Mitnehmers 14 eine Rasteinrichtung 42 (Fig. 25) bilden. Wie Fig. 9 schematisch zeigt, besitzt jeder Raststeg 43 eine radial außen liegende Rastkante 45. Im Ausführungsbeispiel sind die Rastkanten 45 gegenüber der Längsmittelachse 50 um einen Winkel α geneigt. Der Winkel α ist auf den gewünschten Betätigungsweg zwischen der langsamsten und der schnellsten einstellbaren Injektionsgeschwindigkeit und die gewünschte Differenz zwischen der langsamsten und der schnellsten Injektionsgeschwindigkeit abgestimmt. Wie Fig. 9 schematisch zeigt, sind die Raststege 43 an einem Stiftabschnitt 48 des Bedienelements 6 angeordnet, der innerhalb des Hülsenabschnitts 49 und mit radialem Abstand zum Hülsenabschnitt 49 verläuft. An den Stiftabschnitt 48 schließt am proximalen Ende der Verbindungsstutzen 66 an. Im Ausführungsbeispiel sind die Raststege 43 einteilig mit dem Bedienelement 6 ausgebildet und bestehen aus dem gleichen Material wie das Bedienelement 6. Es kann jedoch auch vorteilhaft sein, zur Einstellung einer gewünschten Rastcharakteristik die Raststege 43 aus einem anderen Material auszubilden, beispielsweise aus einem Elastomer oder Gummi.

Wie die Figuren 10 und 11 zeigen, ist das Bedienelement 6 aus einem ersten Einzelteil 39 und einem zweiten Einzelteil 40 aufgebaut, um die Fertigung und Montage zu vereinfachen. Das Bedienelement 6 kann auch aus einer größeren Anzahl von Einzelteilen gebildet sein. Die Einzelteile 39 und 40 sind im Ausführungsbeispiel an einem Rastrand 37 des ersten Einzelteils 39 fest miteinander verbunden.

Die Fig. 12 bis 16 zeigen die Gestaltung des Mitnehmers 14 im Einzelnen. Der Mitnehmer 14 besitzt einen Lagerabschnitt 57 mit verringertem Durchmesser, mit dem der Mitnehmer 14 drehbar im oberen Gehäuseteil 3 gelagert ist. Wie Fig. 13 zeigt, besitzt der Mitnehmer 14 in seinem Inneren einen im proximalen Bereich angeordneten konischen Abschnitt 61, an dessen distaler Seite sich ein zylindrischer Abschnitt 62 anschließt. Der konische Abschnitt 61 und der zylindrische Abschnitt 62 werden durch eine Vielzahl von in den Fig. 14 bis 16 gezeigten Stegen 63 gebildet, die von einer zylindrischen Außenwand 68 des Mitnehmers 14 radial nach innen ragen. In Umfangsrichtung zwischen den Stegen 63 sind die Rastvertiefungen 44 gebildet. Die Stege 63 besitzen radial innen liegende Gegenrastkanten 46, die gegenüber der Längsmittelachse 50 um einen Winkel β geneigt sind. Der Winkel β öffnet dabei in proximaler Richtung. Der Winkel β ist insbesondere gleich groß wie der Winkel α der Rastkanten 45 der Raststege 43.

Im zylindrischen Abschnitt 62 begrenzen die Stege 63 einen Innenraum 60, dessen in Fig. 14 gezeigter Innendurchmesser h nur geringfügig größer als der Außendurchmesser des Stiftabschnitts 48 ist. Die Stege 63 besitzen im zylindrischen Abschnitt 62 eine radial gemessene Höhe f₁, die etwa der radialen Erstreckung der Raststege 43 entspricht.

Fig. 15 zeigt einen Schnitt durch den konischen Abschnitt 61. In der in Fig. 15 gezeigten Schnittebene besitzen die radial innen liegenden Rastkanten 46 der Stege 63 zur Längsmittelachse 50 einen Abstand g₁. Die radial gemessene Höhe f₂ der Stege 63 ist in der in Fig. 15 gezeigten Schnittebene durch den konischen Abschnitt 61 deutlich kleiner als die Höhe f₁ im zylindrischen Abschnitt 62.

Fig. 16 zeigt einen Schnitt durch den konischen Abschnitt 61 benachbart zum proximalen Ende des Innenraums 60. In dieser Schnittebene besitzen die Rastkanten 46 zur Längsmittelachse 50 einen Abstand g₂, der deutlich größer als der Abstand g₁ ist. Die radial gemessene Höhe f₃ der Stege 63 ist in der in Fig. 15 gezeigten Schnittebene durch den konischen Abschnitt 61 deutlich kleiner als die Höhe f₂ in der in Fig. 15 gezeigten, distal liegenden Schnittebene.

Die Fig. 22 bis 29 zeigen Bedienelement 6 und Mitnehmer 14 in unterschiedlichen axialen Relativpositionen. Die Fig. 22 und 23 zeigen Bedienelement 6 und Mitnehmer 14 in einer Kupplungsposition 51. In dieser Position sind das Bedienelement 6 und der Mitnehmer 14 über die Kupplung 16 drehfest miteinander verbunden. Die Kupplung 16 wird durch die Stege 63 im zylindrischen Abschnitt 62 gebildet. Die Stege 63 ragen bis nahe an den Stiftabschnitt 48 und überlappen die Stege 63 in Umfangsrichtung an ihrer distalen Seite mit einer Eingriffstiefe o. Die Eingriffstiefe o ist so gewählt, dass Mitnehmer 14 und Bedienelement 6 drehfest miteinander verbunden sind. Die Fig. 23, 25, 27 und 29 zeigen dabei Schnitte durch das Bedienelement 6 an der distalen Seite der Raststege 43. Der Hülsenabschnitt 49 besitzt eine proximale Stirnseite 65. Die proximale Stirnseite 65 besitzt in der Kupplungsposition 51 zu einer Unterkante 86 des Mitnehmers 14 einen in Richtung der Längsmittelachse 50 gemessenen ersten Abstand n₁. Die Unterkante 86 ist dabei die Kante des Mitnehmers 14, die an dem nach innen ragenden Rand des oberen Gehäuseteils 3 aufliegt.

Die Fig. 24 und 25 zeigen Bedienelement 6 und Mitnehmer 14 in einer ersten Relativposition 52, in der die Einstellvorrichtung 41 zwischen dem Bedienelement 6 und dem Mitnehmer 14 wirkt. Die proximale Stirnseite 65 des Hülsenabschnitts 49 besitzt zur Unterkante 86 des Mitnehmers 14 einen Abstand n₂, der kleiner ist als der Abstand n₁. Wie Fig. 25 zeigt, befinden sich die Raststege 43 im konischen Abschnitt 61, in dem der Abstand der Rastkanten 46 der Stege 63 zum Stiftabschnitt 48 und zur Längsmittelachse 50 verringert ist. In dieser Relativposition überlappen die Raststege 43 die Stege 63 in radialer Richtung an der distalen Seite der Raststege 43 mit einer Rasttiefe m₁. Die Rasttiefe m₁ ist deutlich kleiner als die Eingriffstiefe o in der Kupplungsposition 51. Die Rasttiefe m₁ ist so gewählt, dass sich der Mitnehmer 14 gegenüber dem Bedienelement 6 unter Verformung der Raststege 43 drehen kann.

Um aus der Kupplungsposition 51 in die erste Relativposition 52 zu gelangen, muss der Bediener das Bedienelement 6 in proximaler Richtung 31 bewegen, wie in Fig. 24 angedeutet. Dadurch verschiebt sich das Bedienelement 6 relativ zum oberen Gehäuseteil 3 und die Stege 38 am Bedienelement 6 gelangen in Eingriff mit einer in den Fig. 36 und 38 gezeigten Verrastung 84 an der Innenseite des oberen Gehäuseteils 3. Die Verrastung 84 besitzt eine Vielzahl von Rastelementen 85, die das Bedienelement 6 drehfest gegenüber dem äußeren Gehäuseteil 3 sichern. Die Stege 38 bilden mit der Verrastung 84 die Kupplung 20.

Bei der in den Fig. 24 und 25 gezeigten ersten Relativposition 52 kann sich der Mitnehmer 14 relativ zum oberen Gehäuseteil 3 und zum Bedienelement 6 drehen, wenn die in der Feder 9 gespeicherte Energie ausreichend ist, um die Raststege 43 zu verformen sowie den Stopfen 10 in proximale Richtung zu schieben, so dass die Injektionsflüssigkeit aus dem Behälter 5 ausgepresst wird. Das Auspressen von Injektionsflüssigkeit erfolgt jedoch aufgrund des hohen Drehmoments, das für eine Drehung des Mitnehmers 14 gegenüber dem Bedienelement 6 benötigt wird, sehr langsam.

Bei der in den Fig. 26 und 27 gezeigten zweiten Relativposition 53 von Bedienelement 6 und Mitnehmer 14 hat der Bediener das Bedienelement 6 weiter in proximaler Richtung 31 in das obere Gehäuseteil 3 gedrückt. Die proximale Stirnseite 65 besitzt in dieser Position einen dritten Abstand m₃ zur Unterkante 86 des Mitnehmers 14. Der dritte Abstand m₃ ist deutlich kleiner als der zweite Abstand m₂. Beim Bewegen des Bedienelements 6 in proximale Richtung 31 haben sich die Raststege 43 im konischen Abschnitt 61 weiter in proximale Richtung, also in Richtung auf einen vergrößerten Innendurchmesser des konischen Abschnitts 61 bewegt. Die distale Seite der Raststege 43 besitzt in radialer Richtung zur Längsmittelachse 50 eine geringe Überlappung zu den Stegen 63, so dass sich nur eine geringe Rasttiefe m₂ ergibt. In dieser Position der Einstellvorrichtung 41 ist das für eine Drehung des Mitnehmers 14 gegenüber dem Bedienelement 6 notwendige Drehmoment deutlich kleiner als bei der in den Fig. 24 und 25 gezeigten ersten Relativposition 52. Die Raststege 43 müssen nur geringfügig verformt werden, um die Stege 63 zu überwinden und in die nächste Rastposition zu gelangen. Dadurch erfolgt eine Injektion in der in Fig. 26 und 27 gezeigten zweiten Relativposition mit höherer Geschwindigkeit als bei der in Fig. 24 und 25 gezeigten ersten Relativposition.

Die Fig. 28 und 29 zeigen Bedienelement 6 und Mitnehmer 14 in einer dritten Relativposition 54, in der die Unterkante 86 zur proximalen Stirnseite 65 nur einen sehr geringen vierten Abstand n₄ aufweist. Das Bedienelement 6 befindet sich in der dritten Relativposition 54 in seiner proximalen Endposition, in der der in Fig. 2 gezeigte Absatz 32 am Rand 33 des oberen Gehäuseteils 3 anliegt. Wie Fig. 29 zeigt, besitzen die Raststege 43 in der dritten Relativposition 54 eine äußerst geringe Überlappung in radialer Richtung zu den Stegen 63. Die Rasttiefe m₃ ist minimal. Es kann auch vorgesehen sein, dass die Rasttiefe m₃ null ist, so dass die Raststege 43 sich gegenüber den Stegen 63 frei drehen können und die Einstellvorrichtung 41 in der dritten Relativposition 54 die Drehung des Mitnehmers 14 gegenüber dem Bedienelement 6 nicht bremst. In der dritten Relativposition 54 ergibt sich deshalb die größte mögliche Injektionsgeschwindigkeit. Die Energie, die zur Drehung des Mitnehmers 14 gegenüber dem Bedienelement 6 benötigt wird, ist gering. Die Injektionsgeschwindigkeit wird von der in der Feder 9 gespeicherten Kraft und von den Reibkräften, die zwischen den zueinander bewegten Bauteilen wirken, bestimmt.

Im Ausführungsbeispiel sind der Winkel α der Rastkante 45 des Raststegs 43 und der Winkel β der Rastkante 46 des Stegs 63 gleich. Dadurch ergibt sich über die gesamte Höhe c der Raststege 43 die gleiche Rasttiefe m₁, m₂, m₃. Bei unterschiedlichen Winkeln α, β ergeben sich unterschiedliche Rasttiefen m in unterschiedlichen Abschnitten des Raststegs 43. Die von der Einstellvorrichtung 41 ausgeübte Kraft kann durch geeignete Wahl der Gestalt und Anzahl der Raststege 43 beeinflusst werden.

Die Fig. 30 und 31 zeigen das Dosierorgan 18 im Einzelnen. Das Dosierorgan 18 besitzt ein Außengewinde 75, das mit einem in Fig. 34 gezeigten Innengewinde 80 der Injektionshülse 17 die erste Gewindeverbindung 19 bildet. Das Dosierorgan 18 besitzt einen Lagerstutzen 69, mit dem das Dosierorgan 18 in einer Lageröffnung 82 im oberen Gehäuseteil 3 (Fig. 36 und 37) drehbar gelagert ist. Das Dosierorgan 18 besitzt Anlagestege 76, die an der distalen Seite der Gehäusewand 87, die die Lageröffnung 82 aufweist, anliegen. Dadurch wird die Reibung zwischen dem Dosierorgan 18 und dem oberen Gehäuseteil 3 bei der Drehbewegung des Dosierorgans 18 verringert. Wie Fig. 31 zeigt, ist im Lagerstutzen 69 ein Innengewinde 77 ausgebildet, das mit dem Außengewinde 72 der Kolbenstange 12 die zweite Gewindeverbindung 22 bildet (Fig. 2).

Wie Fig. 32 zeigt, besitzt die Injektionshülse 17 Aussparungen 78, die zur Gewichtsreduzierung dienen. In den Fig. 32 bis 34 ist auch die Öffnung 26 gezeigt, hinter der die Skala 59 des Dosierorgans 18 (Fig. 30) für den Bediener sichtbar ist.

Die Injektionshülse 17 besitzt an ihrem Außenumfang zwei einander gegenüberliegend angeordnete Führungsnuten 79. Im oberen Gehäuseteil 3 sind entsprechende Führungsstege 81 ausgebildet, von denen in Fig. 36 einer sichtbar ist. Die Führungsstege 81 ragen in die Führungsnuten 79 und fuhren dadurch die Injektionshülse 17 im oberen Gehäuseteil 3 in Richtung der Längsachse 50 beweglich und drehfest.

Wie Fig. 37 zeigt, weist die Gehäusewand 87 neben der Lageröffnung 82 eine Durchtrittsöffnung 83 auf, durch die der Steg 34 der Injektionshülse 17 ragt. Der Steg 34 erstreckt sich nicht über den gesamten Umfang der Injektionshülse 17, sondern lediglich über den Umfangsbereich, in dem das Sichtfenster 7 angeordnet ist. Durch den Steg 34 wird eine geringe Baulänge des Injektionsgeräts 1 bei ausreichend großem Verstellbereich für das Dosierorgan 18 erreicht. Beim Einstellen der Dosis dreht sich das Dosierorgan 18, und die Injektionshülse 17 wird in axialer Richtung verschoben. Dadurch ist durch die Öffnung 26 jeweils die eingestellte Dosis sichtbar.

Wie Fig. 38 zeigt, sind die Rastelemente 85 der Verrastung 84 im Ausführungsbeispiel unsymmetrisch ausgebildet. Die Rastelemente 85 wirken mit den Rastelementen 47 am Bedienelement 6 (Fig. 5) zusammen. Aufgrund der unsymmetrischen Ausbildung der Rastelemente kann eine einmal eingestellte Dosis nicht verringert werden. Durch entsprechende Gestaltung der Rastelemente 85 und Rastelemente 47 kann jedoch auch ein Zurückdrehen des Bedienelements 6 zur Verringerung einer eingestellten Dosis möglich sein.

Über die Rastelemente 47 und 85 sind beim Einstellen einer auszupressenden Menge an Injektionsflüssigkeit Klicks hörbar. Da beim Auspressen einer Menge an Injektionsflüssigkeit das Bedienelement 6 und das obere Gehäuseteil 3 drehfest miteinander verbunden sind, sind die Klicks der Rasteinrichtung 35 beim Auspressen von Injektionsflüssigkeit nicht hörbar. Stattdessen sind beim Auspressen von Injektionsflüssigkeit Klicks der Rasteinrichtung 42 der Einstellvorrichtung 41 für den Bediener hörbar. Dabei erzeugt die Rasteinrichtung 42 in der ersten Rastposition 52 lautere Klicks mit größerem zeitlichem Abstand. Je weiter das Bedienelement 6 in proximale Richtung 31 gedrückt wird, umso leiser werden die Klicks und umso schneller folgen die Klicks aufeinander. Dadurch ist für den Bediener die Injektionsgeschwindigkeit hörbar. Die Rastelemente 85 bilden beim Einstellen der auszupressenden Dosis an Injektionsflüssigkeit mit den Rastelementen 47 die Rasteinrichtung 35. Beim Auspressen von Injektionsflüssigkeit wirken die Rastelemente 85 mit den Stegen 38 zusammen und bilden mit diesen die Kupplung 20 und verbinden das Bedienelement 6 drehfest mit dem oberen Gehäuseteil 3.

Die Fig. 39 bis 54 zeigen ein Ausführungsbeispiel eines Injektionsgeräts 101, dessen Aufbau im Wesentlichen dem des Injektionsgeräts 1 aus den vorangegangenen Figuren entspricht. Gleiche Bezugszeichen bezeichnen in allen Figuren einander entsprechende Bauteile. Das Injektionsgerät 101 besitzt ein Gehäuse 2 mit einem Sichtfenster 7, durch das die Injektionshülse 17 sichtbar ist. Das Injektionsgerät 101 besitzt ein Bedienelement 106, das sich in der in den Fig. 39 und 40 gezeigten Nullstellung 28 in einer distalen Endlage 90 befindet. In dieser Lage kann das Bedienelement 6 beispielsweise durch einen nicht gezeigten, an einem Mitnehmer 114 ausgebildeten Anschlag gehalten sein.

Wie Fig. 40 zeigt, unterschiedet sich das Injektionsgerät 101 vom Injektionsgerät 1 im Wesentlichen durch die Gestaltung des Bedienelements 106 und des Mitnehmers 114. Das Bedienelement 106 ist von einer Feder 23 in seine distale Endlage 90 vorgespannt. Zwischen dem Bedienelement 106 und dem oberen Gehäuseteil 3 ist ein Anschlag gebildet, der von einem Absatz 32 des Bedienelements 106 und einem Rand 33 des oberen Gehäuseteils 3 gebildet wird. Zwischen dem Bedienelement 106 und dem oberen Gehäuseteil 3 wirkt eine Rasteinrichtung 35. Der Mitnehmer 114 ist mit einem Drehlager 15 im oberen Gehäuseteil 3 gelagert. Zwischen dem Mitnehmer 114 und dem Bedienelement 106 ist eine Kupplung 116 gebildet, die in der gezeigten distalen Endlage 90 des Bedienelements 106 das Bedienelement 106 mit dem Mitnehmer 114 drehfest verbindet.

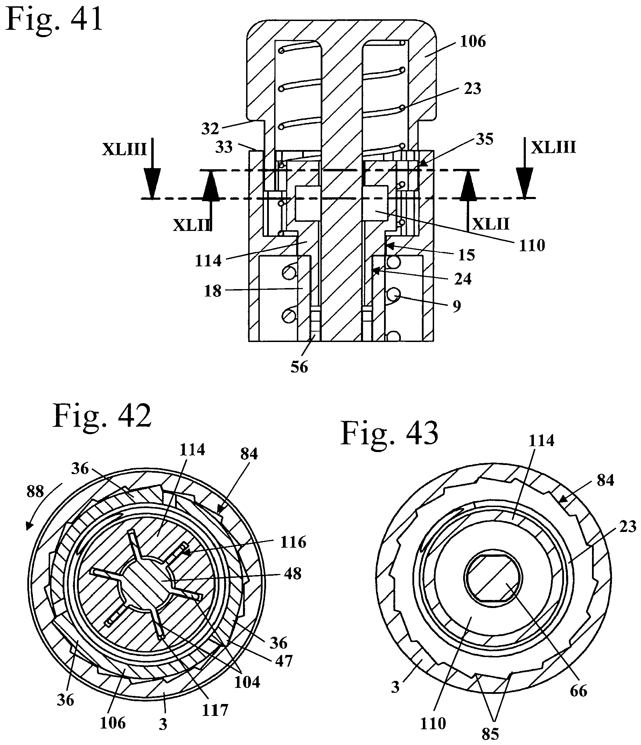

Die Fig. 41 bis 43 zeigen die Gestaltung von Bedienelement 106 und Mitnehmer 114 im Einzelnen. Wie Fig. 42 zeigt, besitzt das obere Gehäuseteil 3 in seinem distalen Endbereich eine Verrastung 84, die durch eine Vielzahl von Rastelementen 85 gebildet ist. Beim Einstellen einer auszupressenden Menge an Injektionsflüssigkeit ist das Bedienelement 106 in einer ersten Drehrichtung 88 gegenüber dem oberen Gehäuseteil 3 zu drehen. Dabei rasten die Rastelemente 47 an den Rastelementen 85 des oberen Gehäuseteils 3 ein. Dabei entstehen für den Bediener hörbare Klickgeräusche. Der Mitnehmer 114 besitzt Aufnahmen 117, die als radial ausgerichtete Schlitze ausgebildet sind und in die Kupplungsstege 104 des Bedienelements 106 ragen. Die Kupplungsstege 104 können entsprechend zu den Raststegen 43 des Injektionsgeräts 1 ausgebildet sein. Die Kupplungsstege 104 bilden mit den Aufnahmen 117 die Kupplung 116.

Wie die Fig. 41 und 43 zeigen, ist an der proximalen Seite des die Aufnahmen 117 aufweisenden Abschnitt des Mitnehmers 114 ein Innenraum 110 gebildet, durch den in der gezeigten distalen Endlage 90 des Bedienelements 106 lediglich der Verbindungsstutzen 66 des Bedienelements 106 ragt.

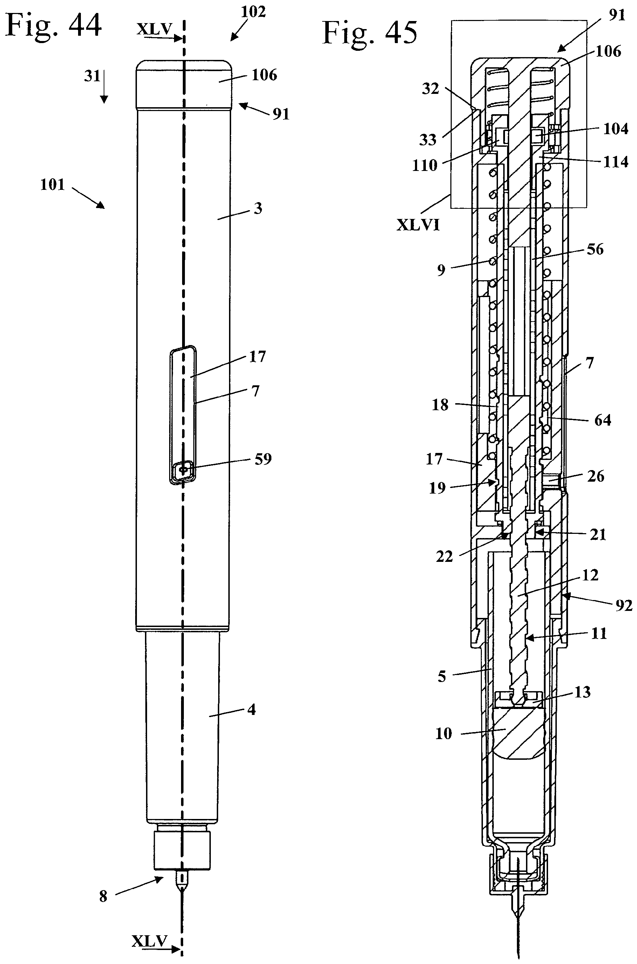

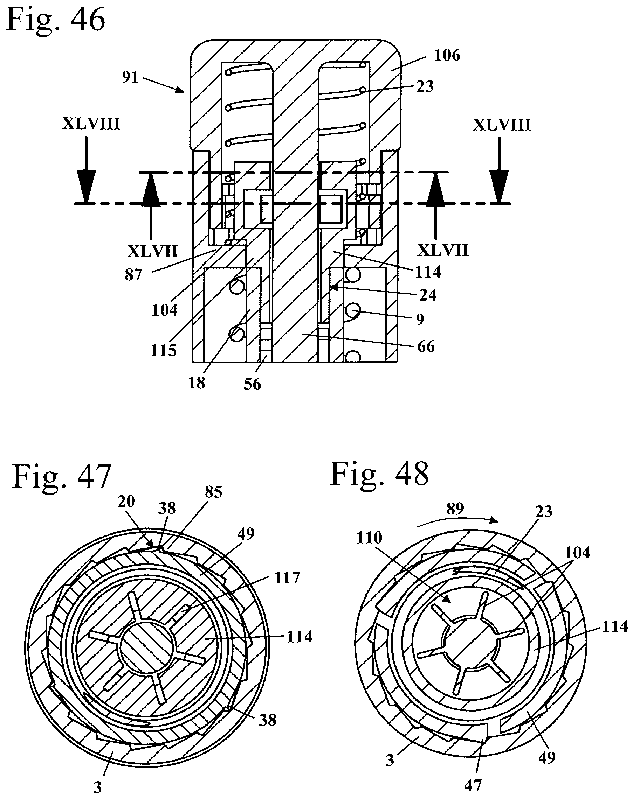

In Fig. 44 und 45 ist das Injektionsgerät 101 in einer Endstellung 102 gezeigt. Nach dem Einstellen einer auszupressenden Menge an Injektionsflüssigkeit durch Drehen des Bedienelements 106 in der ersten Drehrichtung 88 wurde das Bedienelement 106 in proximale Richtung 31 bewegt, bis es in seiner in den Fig. 44 und 45 gezeigten proximalen Endlage 91 zu liegen kam. Bei der proximalen Bewegung des Bedienelements 106 wurde die Kupplung 116 gelöst und der Mitnehmer 114 wurde freigegeben und konnte sich mit dem Dosierorgan 18 um die Längsmittelachse 50 drehen. Die Drehbewegung wurde durch die in der Feder 9 gespeicherte Energie, die die Injektionshülse 17 in proximale Richtung 31 bewegt hat, veranlasst. In der proximalen Endlage 91 des Bedienelements 106 liegt der Absatz 32 des Bedienelements 106 am Rand 33 des oberen Gehäuseteils 3 an und bildet den Anschlag für die proximale Endlage 91. Die Kupplungsstege 104 des Bedienelements 106 sind im Innenraum 110 des Mitnehmers 114 angeordnet. Wie die Fig. 46 und 48 zeigen, können sich die Kupplungsstege 104 im Innenraum 110 frei drehen. Dadurch konnte sich der Mitnehmer 114 mit dem Dosierorgan 18 nach dem Lösen der Kupplung 116 in der zweiten Drehrichtung 89 drehen und dadurch die eingestellte Dosis an Injektionsflüssigkeit aus dem Behälter 5 auspressen.

Wie Fig. 47 zeigt, liegen in der proximalen Endlage 91 des Bedienelements 106 die Stege 38 des Bedienelements 106 zwischen den Rastelementen 85 und verbinden dadurch das Bedienelement 106 drehfest mit dem oberen Gehäuseteil 3. Die Kupplungsstege 104 befinden sich nicht in den Aufnahmen 117, sondern vollständig im Innenraum 110, so dass eine freie Drehung möglich ist. Die Stege 38 bilden mit den Rastelementen 85 der Verrastung 84 die Kupplung 20.

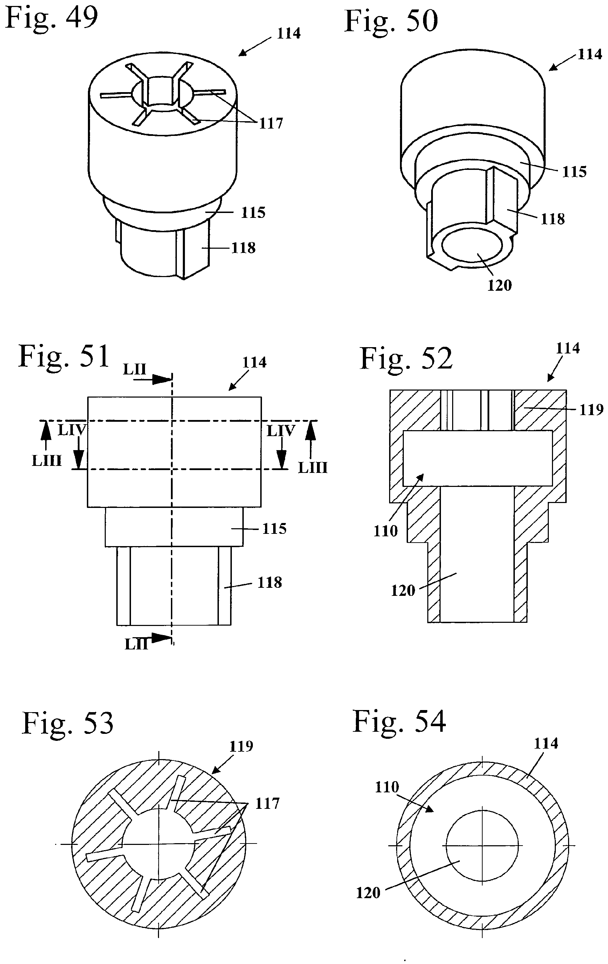

Die Fig. 49 bis 54 zeigen die Gestaltung des Mitnehmers 114 im Einzelnen. Wie Fig. 49 zeigt, sind die Aufnahmen 117 als radial verlaufende Schlitze ausgebildet. Der Mitnehmer 114 besitzt einen Lagerabschnitt 115, mit dem er in der Gehäusewand 87 drehbar gelagert ist (Fig. 46). Wie die Fig. 49 und 50 zeigen, weist der Mitnehmer 114 in einem proximalen Endbereich Sicherungsstege 118 auf, die zur drehfesten Verbindung mit dem Dosierorgan 18 dienen. Über die Sicherungsstege 118 ist der Mitnehmer 114 formschlüssig mit dem Dosierorgan 18 verbunden. Mittig besitzt der Mitnehmer 114 eine Öffnung 120, durch die der Verbindungsstutzen 66 des Bedienelements 106 ragt. Wie die Fig. 52 und 53 zeigen, besitzt der Mitnehmer 114 einen Kupplungsabschnitt 119, in dem die Aufnahmen 117 der Kupplung 116 ausgebildet sind. In dem an der proximalen Seite an den Kupplungsabschnitt 119 anschließenden Innenraum 110 ist der freie Innendurchmesser so groß gewählt, dass die Kupplungsstege 104 (Fig. 46) sich im Innenraum 110 frei drehen können.

Bei dem in den Fig. 39 bis 54 gezeigten Ausführungsbeispiel eines Injektionsgeräts 101 ist die Injektionsgeschwindigkeit durch den Bediener nicht wählbar, sondern durch die in der Feder 9 gespeicherte Energie, die Viskosität der Injektionsflüssigkeit, des Durchmessers der Injektionsnadel und die Reibung des Stopfens 10 gegenüber dem Behälter 5 bestimmt. Beim Injektionsgerät 1 kann die Injektionsgeschwindigkeit dagegen durch den Bediener durch entsprechende Positionierung des Bedienelementes 6 gewählt werden.

## Patentansprüche

1. Injektionsgerät mit einem Gehäuse (2), das eine Längsmittelachse (50) besitzt, mit einem drehbar und in Richtung der Längsmittelachse (50) fest im Gehäuse (2) gehaltenen Dosierorgan (18), das über eine erste Gewindeverbindung (19) mit einer gegenüber dem Gehäuse (2) drehfest und in Richtung der Längsmittelachse (50) verschiebbar gehaltenen Injektionshülse (17) verbunden ist, wobei sich das Dosierorgan (18) beim Einstellen einer auszupressenden Dosis an Injektionsflüssigkeit gegenüber dem Gehäuse (2) dreht und sich die Injektionshülse (17) aufgrund der ersten Gewindeverbindung (19) in distale Richtung (30) bewegt und wobei sich das Dosierorgan (18) beim Auspressen einer eingestellten Dosis an Injektionsflüssigkeit in Gegenrichtung gegenüber dem Gehäuse (2) dreht und sich die Injektionshülse (17) aufgrund der ersten Gewindeverbindung (19) in proximale Richtung (31) bewegt, wobei das Injektionsgerät (1) eine Feder (9) besitzt, die sich mit einem ersten Ende (70) gegenüber der Injektionshülse (17) und mit einem zweiten Ende (71) gegenüber dem Gehäuse (2) abstützt und die die Injektionshülse (17) beim Auspressen von Injektionsflüssigkeit in proximale Richtung (31) bewegt und dadurch das Auspressen der eingestellten Dosis von Injektionsflüssigkeit bewirkt, wobei das Injektionsgerät (1, 101) einen Dosierkolben (11) zum Auspressen von Injektionsflüssigkeit aus dem Behälter (5) besitzt,
**dadurch gekennzeichnet, dass** der Dosierkolben (11) über eine zweite Gewindeverbindung (22) mit dem Dosierorgan (18) verbunden ist, wobei der Dosierkolben (11) beim Einstellen der auszupressenden Menge an Injektionsflüssigkeit drehfest mit dem Dosierorgan (18) verbunden ist und sich mit dem Dosierorgan (18) dreht, und wobei der Dosierkolben (11) beim Auspressen einer auszupressenden Menge an Injektionsflüssigkeit drehfest mit dem Gehäuse (2) verbunden ist und sich aufgrund der zweiten Gewindeverbindung (22) in proximale Richtung (31) bewegt.

2. Injektionsgerät nach Anspruch 1,
**dadurch gekennzeichnet, dass** die Feder (9) als Druckfeder ausgebildet ist.

3. Injektionsgerät nach Anspruch 1 oder 2,
**dadurch gekennzeichnet, dass** die Feder (9) sich mit ihrem ersten Ende (70) an der Injektionshülse (17) und mit ihrem zweiten Ende (71) am Gehäuse (2) abstützt.

4. Injektionsgerät nach einem der Ansprüche 1 bis 3,
**dadurch gekennzeichnet, dass** das Dosierorgan (18) radial innerhalb der Injektionshülse (17) angeordnet ist.

5. Injektionsgerät nach Anspruch 4,
**dadurch gekennzeichnet, dass** die Feder (9) am Außenumfang des Dosierorgans (18) und mindestens teilweise in einem zwischen Dosierorgan (18) und Injektionshülse (17) gebildeten Ringraum (64) angeordnet ist.

6. Injektionsgerät nach Anspruch 4 oder 5,
**dadurch gekennzeichnet, dass** das Gehäuse (2) ein Sichtfenster (7) besitzt, dass die Injektionshülse (17) eine Öffnung (26) aufweist, die in Überdeckung mit dem Sichtfenster (7) liegt und durch die eine am Außenumfang des Dosierorgans (18) angeordnete Skala (59) sichtbar ist, und dass die Injektionshülse (17) einen Abschnitt besitzt, der bei maximaler eingestellter Dosis den proximalen Bereich des Sichtfensters (7) abdeckt.

7. Injektionsgerät nach Anspruch 6,
**dadurch gekennzeichnet, dass** der Abschnitt, der den proximalen Bereich des Sichtfensters (7) abdeckt, an einem in proximale Richtung (31) ragenden Steg (34) der Injektionshülse (17) ausgebildet ist.

8. Injektionsgerät nach Anspruch 7,
**dadurch gekennzeichnet, dass** das Injektionsgerät (1) einen Behälter (5) mit Injektionsflüssigkeit umfasst, und dass in radialer Richtung zwischen dem Behälter (5) und einem oberen Gehäuseteil (3) des Gehäuses (2) eine Tasche (92) gebildet ist, in die der Steg (34) in Nullstellung (28) des Injektionsgeräts (1) ragt.

9. Injektionsgerät nach Anspruch 7 oder 8,
**dadurch gekennzeichnet, dass** das Gehäuse (2) eine Gehäusewand (87) besitzt, an der ein Drehlager (21) für das Dosierorgan (18) ausgebildet ist, und dass der Steg (34) durch eine Durchtrittsöffnung (83) in der Gehäusewand (87) auf die proximale Seite der Gehäusewand (87) ragt.

10. Injektionsgerät nach einem der Ansprüche 1 bis 9,
**dadurch gekennzeichnet, dass** die Injektionshülse (17) in jeder Stellung des Injektionsgeräts (1) vollständig im Gehäuse (2) des Injektionsgeräts (1) angeordnet ist.

11. Injektionsgerät nach einem der Ansprüche 1 bis 10,
**dadurch gekennzeichnet, dass** das Injektionsgerät (1, 101) ein Bedienelement (6, 106) besitzt, das beim Einstellen der auszupressenden Dosis an Injektionsflüssigkeit über eine erste Kupplung (16, 116) drehfest mit dem Dosierorgan (18) verbunden ist und beim Auspressen einer eingestellten Dosis an Injektionsflüssigkeit über eine zweite Kupplung (20) drehfest mit dem Gehäuse (2) verbunden und gegenüber dem Dosierorgan (18) drehbar ist.

12. Injektionsgerät nach Anspruch 11,
**dadurch gekennzeichnet, dass** das Bedienelement (6, 106) sich beim Einstellen der Dosis in einer distalen Endlage (90) befindet und zum Lösen der ersten Kupplung (16, 116) in proximaler Richtung (31) zu bewegen ist.

## Claims

1. An injection device comprising a housing (2), which has a longitudinal central axis (50), and a metering member (18) which is held in the housing (2) so as to be rotatable and fixed in the direction of the longitudinal central axis (50) and which is connected via a first threaded connection (19) to an injection sleeve (17) which is rotationally fixedly held with respect to the housing (2) and is displaceably held in the direction of the longitudinal central axis (50), wherein, on the setting of a dosage of injection liquid to be squeezed out, the metering member (18) rotates with respect to the housing (2) and the injection sleeve (17) moves in the distal direction (30) due to the first threaded connection (19), and wherein, on the squeezing out of a set dosage of injection liquid, the metering member (18) moves in the opposite direction with respect to the housing (2) and the injection sleeve (17) moves in the proximal direction (31) due to the first threaded connection (19), wherein the injection device (1) has a spring (9) which is supported with respect to the injection sleeve (17) with its first end (70) and is supported with respect to the housing (2) with its second end (71) and which moves the injection sleeve (17) in the proximal direction (31) on the squeezing out of injection liquid and thereby brings about the squeezing out of the set dosage of injection liquid, wherein the injection device (1, 101) has a metering piston (11) for squeezing injection liquid out of the container (5),
**characterized in that** the metering piston (11) is connected to the metering member (18) via a second threaded connection (22), with, on the setting of the amount of injection liquid to be squeezed out, the metering piston (11) being rotationally fixedly connected to the metering member (18) and rotating with the metering member (18), and with, on the squeezing out of an amount of injection liquid to be squeezed out, the metering piston (11) being rotationally fixedly connected to the housing (2) and moving in the proximal direction (31) due to the second threaded connection (22).

2. An injection device in accordance with claim 1,
**characterized in that** the spring (9) is configured as a compression spring (9).

3. An injection device in accordance with claim 1 or claim 2,
**characterized in that** the spring (9) is supported at the injection sleeve (17) with its first end (70) and is supported at the housing (2) with its second end (71).

4. An injection device in accordance with any one of the claims 1 to 3,
**characterized in that** the metering member (18) is arranged radially within the injection sleeve (17).

5. An injection device in accordance with claim 4,
**characterized in that** the spring (9) is arranged at the outer periphery of the metering member (18) and is at least partly arranged in an annular space (64) formed between the metering member (18) and the injection sleeve (17).

6. An injection device in accordance with claim 4 or claim 5,
**characterized in that** the housing (2) has a viewing window (7); **in that** the injection sleeve (17) has an opening (26) which superposes the viewing window (7) and through which a scale (59) arranged at the outer periphery of the metering member (18) is visible; and **in that** the injection sleeve (17) has a section which covers the proximal region of the viewing window (7) at the maximum set dosage.

7. An injection device in accordance with claim 6,
**characterized in that** the section which covers the proximal region of the viewing window (7) is formed at a web (34) of the injection sleeve (17) projecting in the proximal direction (31).

8. An injection device in accordance with claim 7,
**characterized in that** the injection device (1) has a container (5) comprising injection liquid; and **in that** a pocket (92), into which the web (34) projects in the zero position (28) of the injection device (1), is formed in the radial direction between the container (5) and an upper housing part (3) of the housing (2).

9. An injection device in accordance with claim 7 or claim 8,
**characterized in that** the housing (2) has a housing wall (87) at which a pivot bearing (21) for the metering member (18) is formed; and **in that** the web (34) projects through a passage opening (26) in the housing wall (87) onto the proximal side of the housing wall (87).

10. An injection device in accordance with any one of the claims 1 to 9,
**characterized in that** the injection sleeve (17) is completely arranged in the housing (2) of the injection device (1) in every position of the injection device (1).

11. An injection device in accordance with any one of the claims 1 to 10,
**characterized in that** the injection device (1, 101) has an operating element (6, 106) which is rotationally fixedly connected to the metering member (18) via a first coupling (16, 116) on the setting of the dosage of injection liquid to be squeezed out and which is rotationally fixedly connected to the housing (2) via a second coupling (20) and rotatable with respect to the metering member (18) on the squeezing out of a set dosage of injection liquid.

12. An injection device in accordance with claim 11,
**characterized in that**, on the setting of the dosage, the operating element (6, 106) is in a distal end position (90) and is to be moved in the proximal direction (31) for the release of the first coupling (16, 116).

## Revendications

1. Dispositif d'injection comportant un boîtier (2) qui présente un axe médian longitudinal (50), un organe de dosage (18) qui est maintenu dans le boîtier (2) de façon mobile en rotation et fixe dans la direction de l'axe médian longitudinal (50) et qui est relié par une première liaison filetée (19) à un manchon d'injection (17) maintenu solidairement en rotation par rapport au boîtier (2) et de façon mobile en translation dans la direction de l'axe médian longitudinal (50),
dans lequel
lors du réglage d'une dose de liquide d'injection à éjecter, l'organe de dosage (18) tourne par rapport au boîtier (2) et le manchon d'injection (17) se déplace dans la direction distale (30) en raison de la première liaison filetée (19), et lors de l'éjection d'une dose réglée de liquide d'injection, l'organe de dosage (18) tourne dans la direction opposée par rapport au boîtier (2) et le manchon d'injection (17) se déplace dans la direction proximale (31) en raison de la première liaison filetée (19),
le dispositif d'injection (1) possède un ressort (9) dont une première extrémité (70) s'appuie contre le manchon d'injection (17) et dont une seconde extrémité (71) s'appuie contre le boîtier (2), et qui, lors de l'éjection du liquide d'injection, déplace le manchon d'injection (17) dans la direction proximale (31) et provoque ainsi l'éjection de la dose réglée de liquide d'injection,
le dispositif d'injection (1, 101) possède un piston de dosage (11) pour éjecter le liquide d'injection hors du récipient (5),
**caractérisé en ce que**
le piston de dosage (11) est relié à l'organe de dosage (18) par une seconde liaison filetée (22), le piston de dosage (11) étant relié solidairement en rotation à l'organe de dosage (18) et tournant avec l'organe de dosage (18) lors du réglage de la quantité de liquide d'injection à éjecter, et, lors de l'éjection d'une quantité de liquide d'injection à éjecter, le piston de dosage (12) étant relié solidairement en rotation au boîtier (2) et se déplaçant dans la direction proximale (31) en raison de la seconde liaison filetée (22).

2. Dispositif d'injection selon la revendication 1,
**caractérisé en ce que** le ressort (9) est réalisé sous forme de ressort de compression.

3. Dispositif d'injection selon la revendication 1 ou 2,
**caractérisé en ce que** le ressort (9) s'appuie par sa première extrémité (70) contre le manchon d'injection (17) et par sa seconde extrémité (71) contre le boîtier (2).

4. Dispositif d'injection selon l'une des revendications 1 à 3,
**caractérisé en ce que** l'organe de dosage (18) est disposé radialement à l'intérieur du manchon d'injection (17).

5. Dispositif d'injection selon la revendication 4,
**caractérisé en ce que** le ressort (9) est disposé sur la circonférence extérieure de l'organe de dosage (18) et au moins partiellement dans un espace annulaire (64) formé entre l'organe de dosage (18) et le manchon d'injection (17).

6. Dispositif d'injection selon la revendication 4 ou 5,
**caractérisé en ce que** le boîtier (2) présente une fenêtre d'observation (7), **en ce que** le manchon d'injection (17) présente une ouverture (26) qui est en chevauchement avec la fenêtre d'observation (7) et à travers laquelle une échelle (59) disposée sur la circonférence extérieure de l'organe de dosage (18) est visible, et **en ce que** le manchon d'injection (17) présente une portion qui recouvre la zone proximale de la fenêtre d'observation (7) lorsque la dose est réglée au maximum.

7. Dispositif d'injection selon la revendication 6,
**caractérisé en ce que** la portion recouvrant la zone proximale de la fenêtre d'observation (7) est formée sur une barrette (34) du manchon d'injection (17) faisant saillie dans la direction proximale (31).

8. Dispositif d'injection selon la revendication 7,
**caractérisé en ce que** le dispositif d'injection (1) comprend un récipient (5) avec un liquide d'injection, et **en ce qu'**une poche (92) est formée dans la direction radiale entre le récipient (5) et une partie supérieure (3) du boîtier (2), poche dans laquelle la barrette (34) fait saillie, en position zéro (28) du dispositif d'injection (1).

9. Dispositif d'injection selon la revendication 7 ou 8,
**caractérisé en ce que** le boîtier (2) possède une paroi de boîtier (87) sur laquelle est réalisé un palier de rotation (21) pour l'organe de dosage (18), et **en ce que** la barrette (34) fait saillie à travers une ouverture de passage (83) dans la paroi de boîtier (87) vers le côté proximal de la paroi de boîtier (87).

10. Dispositif d'injection selon l'une des revendications 1 à 9,
**caractérisé en ce que** le manchon d'injection (17) est entièrement disposé dans le boîtier (2) du dispositif d'injection (1), dans n'importe quelle position du dispositif d'injection (1).

11. Dispositif d'injection selon l'une des revendications 1 à 10,
**caractérisé en ce que** le dispositif d'injection (1, 101) possède un élément de commande (6, 106) qui, lors du réglage de la dose de liquide d'injection à éjecter, est relié solidairement en rotation à l'organe de dosage (18) par un premier accouplement (16, 116) et qui, lors de l'éjection d'une dose réglée de liquide d'injection, est relié solidairement en rotation au boîtier (2) par un second accouplement (20) et est mobile en rotation par rapport à l'organe de dosage (18).

12. Dispositif d'injection selon la revendication 11,
**caractérisé en ce que** lors du réglage de la dose, l'élément de commande (6, 106) est situé dans une position de fin de course distale (90) et est à déplacer dans une direction proximale (31) pour libérer le premier accouplement (16, 116).
